# EUROPEAN PATENT APPLICATION

(11) **EP 2 479 188 A1**
(43) Date of publication of application: **25.07.2012**
(21) Application number: 11151912.0
(22) Date of filing: 24.01.2011
(51) Int. Cl.: C07K 14/37, C12N 9/24

(54) **Endo Tv, a novel high-mannose-specific endo-ß-N-acetylglucosaminidase from trichoderma viride**

(71) Applicant: National University of Ireland, Galway, Galway (IE)
(72) Inventor: Joshi, Lokesh, Moycullen, Galway (IE); Gerlach, Jared, Barna, Galway (IE); Kilcoyne, Michelle, Barna, Co. Galway (IE); Farrell, Mark, Ballinasloe, Co. Galway (IE)
(74) Representative: Gates, Marie Christina Esther

(57) **Abstract**

The invention relates to an *endo*-β-*N*-acetylglucosaminidase enzyme isolatable from the fungus *Trichoderma virde and to methods of producing the enzyme and uses of said enzyme.*

## Description

### Field of the Invention

The present invention relates to a novel *endo*-β-*N*-acetylglucosaminidase isolated from *T*. *virde* (Endo Tv), to a method of producing it and to uses of the novel enzyme preparation.

### Back2round to the Invention

Enzymatic release of N-linked oligosaccharides from glycoproteins is often the preferred method for the analysis of both oligosaccharides and the proteins to which they are attached. The usual enzymes used are amidases (*N*-glycopeptidase, EC 3.5.1.52), such as PNGase A or F, which cleaves the amide bond of carbohydrate linked to an asparagine (Asn) in the protein backbone, or endoglycosidases (*endo*-β-*N*-acetylglucosaminidases, EC 3.2.1.96), such as Endo F₁, F₂, F₃ or H, which hydrolyze the glycosidic bond between the two β-*N-*acetylglucosamine (GlcNAc) residues (also known as the di-*N*-acetylchitobiose core) proximal to the polypeptide (see Table 1). Amidases typically require the denaturation of the protein to allow access of the enzyme to the targeted amide bond. However, some applications require that the protein remain in its native form for downstream analysis (e.g. enzyme activity characterization), while in other applications (e.g. mass spectrometry) maintaining the proximal GlcNAc at the glycosylation site is desirable, and endoglycosidases are preferred.

Endoglycosidases are in high demand for use in glycobiological research and industrial processes. Characteristic activities reported for a variety of endoglycosidases (Table 1) indicate a high degree of selectivity of oligosaccharide structures depending upon the particular enzyme. For example, Endo F₁ can cleave high-mannose type and hybrid biantennary *N*-linked oligosaccharides, while Endo H will cleave oligomannosyl glycans which can also include fucosylation of the proximal GlcNAc. Hydrolysis of fucosylated structures using Endo F₁ is much less efficient. Endoglycosidases which have different specificities would find applications in a variety of research such as particular structure-function studies and scaled-up industrial processes for production and purification of biomolecules.

Chitin is a polymer of β-1,4-linked GlcNAc repeating units and is a component of insect and crustacean exoskeletons and the cell walls of fungi. Chitinases are produced across all classes of organisms, including mammals, and can sometimes also degrade closely related structures of chitin such as chitosan. Filamentous fungi included in the genus Trichoderma (Hyprocrea) are widely exploited in industry for their copious cellulose-degrading extracellular hydrolases. *Trichoderma viride* is known to secrete the chitinases *exo-*GlcNAcase (EC 3.2.1.52) and *endo*-G1cNAcase (EC 3.2.1.14), which degrades both chitin and chitosan. However, there have been no reports describing the ability of *endo-* and *exo-*chitinases to cleave the di-*N*-acetylchitobiose core of Asn-linked oligosaccharide structures. Recently, a novel extracellular *endo*-β-*N*-acetylglucosaminidase from *Trichoderma reesei* (*Hypocrea jecorina*) was reported (16). The *T. reesei* enzyme which is the subject of WO 2006/050584 was not a homologue of bacterial *endo*-β-*N*-acetylglucosaminidases, but rather a very different structure with some conserved features also shared by chitinases from a variety of fungal species. Apart from a report of *endo*-β-*N*-acetylglucosaminidase activity in *Mucor hiemalis* (17), there are no further reported examples of fungal endoglycosidases which act on the di-*N*-chitobiose core of *N*-linked oligosaccharides nor on their preferences for structural isoforms of oligosaccharides.

### Object of the Invention

It is an object of the present invention to provide a novel *endo*-β-*N*-acetylglucosaminidase from *T. viride.* Another object is to provide a method of production and purification of this enzyme. A still further object is to provide an enzyme with a preference for the hydrolysis of certain structural isoforms of high-mannose *N*-linked oligosaccharides of the enzyme as compared to a pure *endo*-β-*N*-acetylglucosaminidase from *Streptomyces plicatus.* It is also an object of the invention to provide an enzyme which does not realease hybrid structures while simultaneously having the ability to release high-mannose structures from glycoproteins. The novel enzyme has been named Endo Tv herein.

### Summary of the Invention

According to the present invention there is provided an *endo*-β-*N*-acetylglucosaminidase enzyme isolatable from the fungus *Trichoderma virde* characterised in that:-
a) the enzyme has an approximate molecular mass of 35 kDa;
b) the enzyme is *endo*-acting;
c) the enzyme can cleave high mannose type *N*-linked oligosaccharides from native bovine RNase B, ovalbumin and yeast invertase, at pH 4.5 to 7 under non-denaturing conditions.
d) the enzyme does not release *N*-linked oligosaccharides containing extended, non-mannosyl residues (i.e. complex) or *N*-linked oligosaccharides containing extended non-mannosyl residues in conjunction with mannosyl extended structures (i.e. hybrid);
e) the Endo Tv enzyme has no activity on fucosylated or bisecting N-acetylglucosamine (GlcNAc)-bearing structures.

The enzyme may be isolatable from organisms other than *Trichoderma virde.* The term "non-denaturing conditions" means that the substrate remains in substantially its native configuration. Preferably the pH for step c is pH 5.0.

Thus in contrast to Endo H from *Streptomyces plicatus,* the Endo Tv enzyme has no activity on fucosylated.

The enzyme, on trypsin digestion, releases all of the following peptides :-
AEPTDLPR, EPARLIAR and LLAVVLSTLLVFGFAPVAK.

The enzyme may result in an increase in the relative percentage release of Man₅GlcNAc and Man₇GlcNAc isomer I and a decrease in the proportion of Man₈GlcNAc isomer I, from RNase B, as compared to the structural isoforms released by WChTv, the commercially available chitinase preparation obtainable from Sigma.

The enzyme may release proportionately more Man₅GlcNAc and Man₇GlcNAc isomer I and less of Man₈GlcNAc isomer I and Man₉GlcNAc than does Endo H.

The enzyme is isolatable from whole chitinase preparation from *Trichoderma virile* (WChTv) or from the medium in which *Trichoderma viride* has been cultured. The enzyme may be induced from *Trichoderma viride* or other organisms. Induction may be achieved by growing the organism in the presence of Chitin. 1% by weight of Chitin in the growth medium would be suitable for induction.

The enzyme has no activity on fucosylated or bisecting *N*-acetylglucosamine-bearing *N-* linked oligosaccharide structures, such as those from ovalbumin, those with xylosyl side modifications, such as those from horseradish proxidase, or upon complex structures such as those from fetuinand immunoglobulin G.

The invention also provides a method for the purification of *endo*-β-*N*-acetylglucosaminidase by any combination of ion exchange chromatography, size exclusion chromatography, hydrophobic interaction chromatography and ammonium sulphate precipitation. For example, a process of weak ion exchange chromatography followed by strong ion exchange and size exclusion chromatography would be suitable to purify the enzymes of the invention.

In a particular embodiment the method of purification comprises :-
a) solubilisation of a whole chitinase preparation from *Trichoderma virde* in an aqueous buffer;
b) purification of the solubilised preparation by size exclusion techniques;
c) selection of at least one fraction which elutes between 195 and 295 mM NaOAc
d) further purification of the selected fraction produced in step (c) by size exclusion chromatography with a salt buffer at pH 4.5 - 7.

The aqueous buffer for solubilisation may be NaOAc. The salt buffer of step (d) may be NaOAc. Step (d) may be carried out in 50mM NaOAc at pH 5.0.

Alternatively, the enzyme may be isolated by filtration and sequential step liquid chromatography of the secreted protein mixture contained in the culture medium of *Trichoderma virde.*

In yet another aspect the invention provides an *endo*-β-*N*-acetylglucosaminidase whenever prepared by a process as described above.

The invention also provides use of an enzyme as defined above to remove oligomannose (M>9 i.e. of more than 9 mannose units) and high-mannose structures (M1-9 i.e. of between 1 and 9 mannose units) from glycoprotein drug products. The glycoprotein drug product may have been produced competently from yeast.

The enzyme may also be used for the quantitative and qualitative analysis of oligomannose and high-mannose components of glycoprotein samples, or to release glycans from fungal glycoproteins, or in a method of verifying the presence of oligomannose and high-mannose structures on glycoprotein. Further uses of the enzyme of the present invention include the removal of mannose from biomaterials so that such molecules do not activate macrophages in the immune system and thus do not induce carbohydrate-mediated immunogenicity. The biomaterials may include bacteria and fungi so that these can be used as vectors. The enzyme could also be used to increase the half life of molecules thus preventing or reducing the rate at which the liver removes mannose from biomolecules. The enzyme also finds use in the synthesis of products by transferase to make glycoprotein structures and to generate high mannose molecules for CDG treatment, since mannose can prevent binding of *E-coli.* The enzyme would also find use in the production of therapeutics, nutraceuticals, and the synthesis of reagents for drug production and analysis.

An enzyme substantially as described herein with reference to the accompanying drawings. The invention also provides a method for the purification of *endo*-β-*N*-acetylglucosaminidase substantially as described herein with reference to the accompanying drawings, and an enzyme whenever produced by a process as defined herein.

### Brief Description of the Drawings

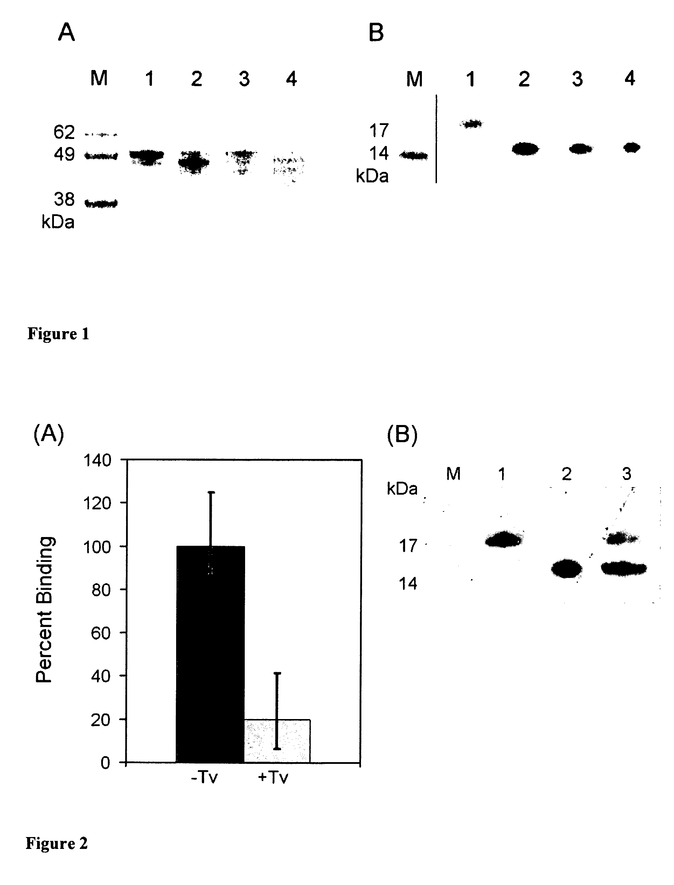

**Fig 1****.** SDS-PAGE of fetuin and RNase B treated with various enzymes. (A) Native fetuin untreated (1), *T. viride* chitinase treated for 2 hours (2), Endo F₂ (3) and Endo F₃ (4). (B) Comparison of (1) untreated RNase B with samples treated with (2) *T. viride* chitinase; (3) Endo H; and (4) Endo F₁. Coomassie G-250 stained gel. Line indicates point where gel image has been cut and spliced together for clarity.
**Figure 2****.** (A) FLLA of ConA-FITC binding to RNase B when untreated (-Tv) and treated (+Tv) with C8241 chitinase preparation from *T. virile.* Untreated sample mean = 100%, *n* = 4. (B) WGA staining of RNase B on PVDF. (1) Untreated RNase B, and RNase B treated with (2) WChTv and (3) WChTvl.

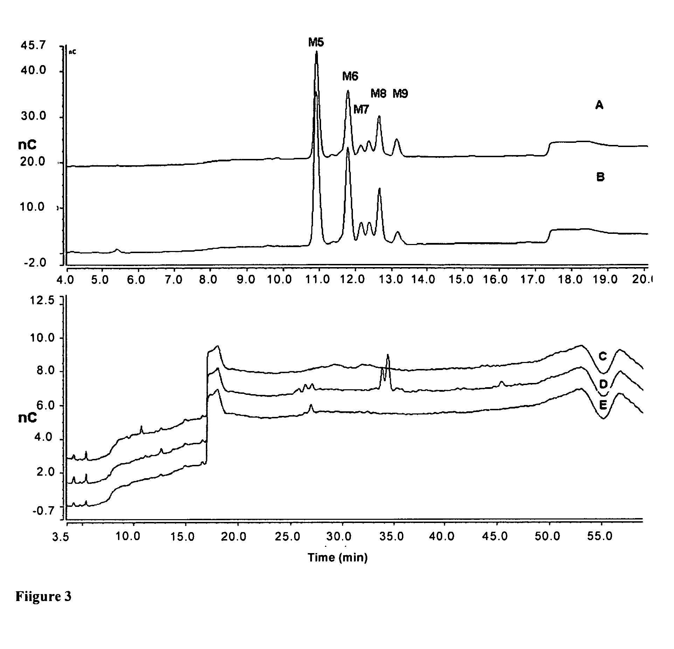

**Figure 3** HPAEC-PAD chromatography of (A) WChTv-released oligosaccharides from RNAse B, where M5 to M9 denote Man₅GlcNAc to Man₉GlcNAc, (B) Endo H-released oligosaccharides from RNAse B, (C) WChTv-released oligosaccharides (none) from fetuin, (D) Endo F₃-released oligosaccharides from fetuin and (E) Endo F₂-released oligosaccharides.

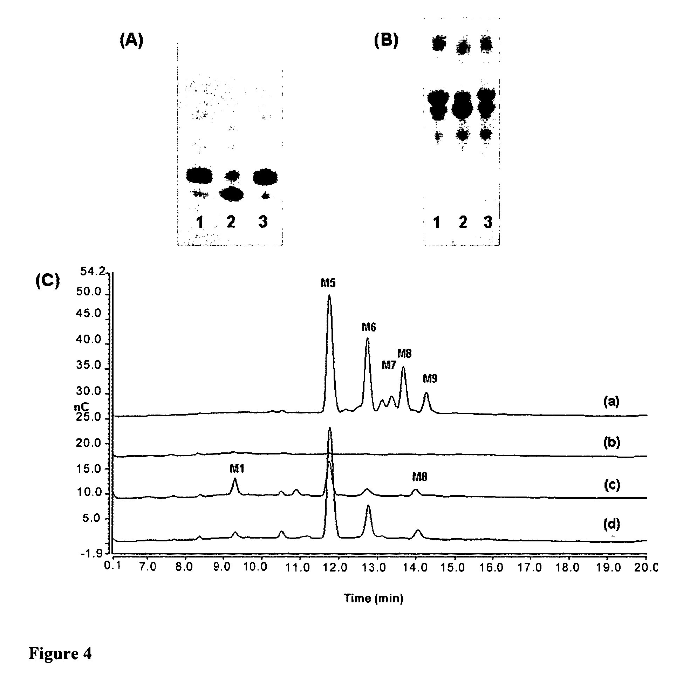

**Figure 4 (A)** Coomassie stained SDS-PAGE of lane 1, RNase B, lane 2, WChTv-treated RNase B and lane 3, WChSg-treated RNase B. (**B**) Coomassie stained SDS-PAGE of lane 1, ovalbumin, lane 2, WChTv-treated ovalbumin and lane 3, WChSg-treated ovalbumin. (**C**) HPAEC-PAD chromatography of released products from (a) Endo H-digested RNase B, where M5 to M9 denotes Man₅GlcNAc to Man₉GlcNAc oligosaccharides, (b) WChSg-treated ovalbumin, (c) WChTv-treated ovalbumin and (d) Endo H-treated ovalbumin.

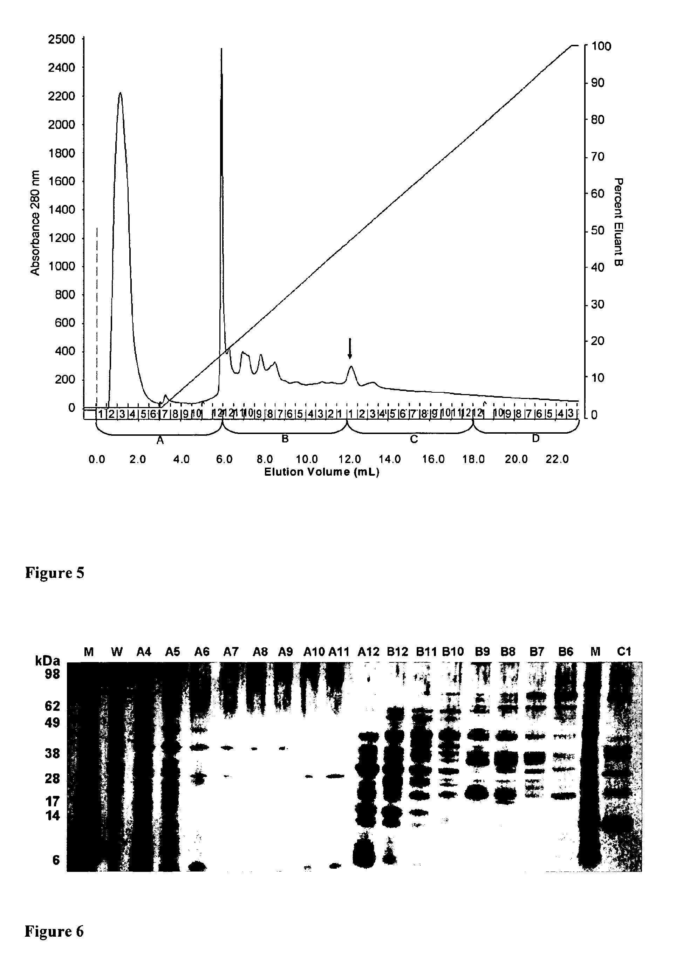

**Figure 5**. Chromatogram from anion exchange separation of filtered protein originating from 8.0 mg chitinase preparation solids. Arrow indicates fraction which demonstrated deglycosylation activity on RNase B.
**Figure 6**. Silver stained SDS-PAGE of fractions produced by anion exchange separation of 650 µg soluble protein from WChTv. WChTv is indicated by W, molecular weight markers indicated by M. Fraction C1 was further purified by size exclusion as follows.

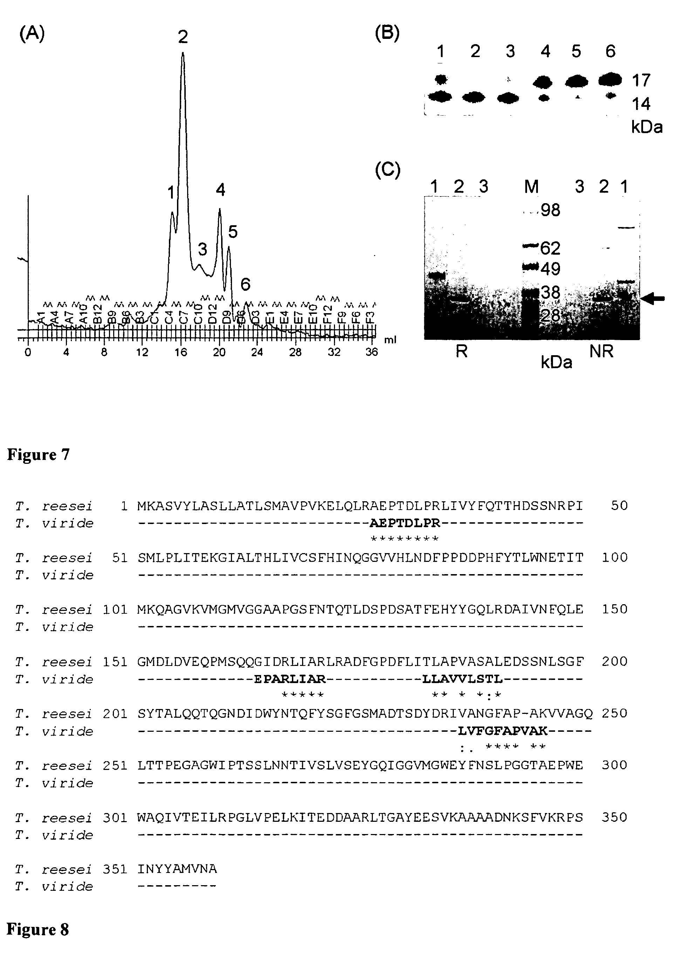

**Figure 7**. (A) Chromatogram of fraction C1 separated on Superdex 200 10/300 size exclusion column. Numbers 1-6 indicated fractions screened for endoglycosidase activity with RNase B. (B) RNase B samples incubated for 2 h 5 uL of indicated fractions from size exclusion. (C) SDS-PAGE of protein from 50 uL of fractions 1, 2 and 3 under reducing (R) and nonreducing (NR) conditions. Arrow indicates band sequenced and described in following text.
**Figure 8**. ClustalW alignment of peptides from nLC-MS/MS analysis of *T. viride endo*-β-*N-*acetylglucosaminidase (bold) and the reported sequence for *endo*-β-*N*-acetylglucosaminidase from *T. reesei.* Asterisks (*) under sequences indicate identically-conserved residues while double dots (:) indicate conserved substitutions and singe dots (˙) indicate semi-conserved substitutions.

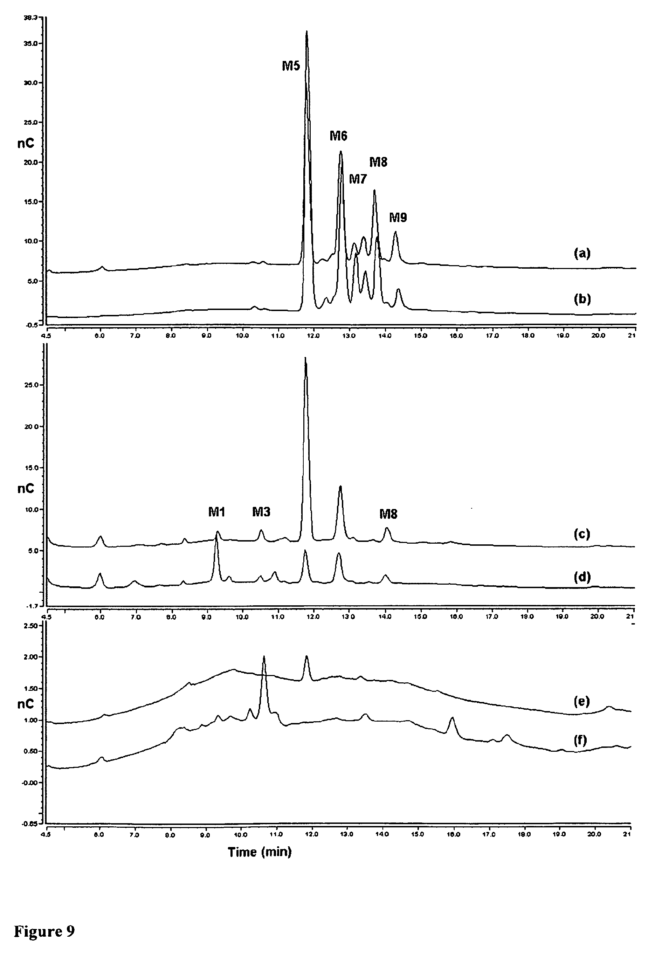

**Figure 9** HPAEC-PAD chromatography of released products purified from (a) Endo H-digested RNase B, where M5 to M9 denote Man₅GlcNAc to Man₉GlcNAc oligosaccharides, (b) Endo Tv-digested RNase B, (c) Endo H-digested ovalbumin, (d) Endo Tv-digested ovalbumin, and (e) Endo Tv-digested fetuin, (f) Endo Tv-digested HRP.

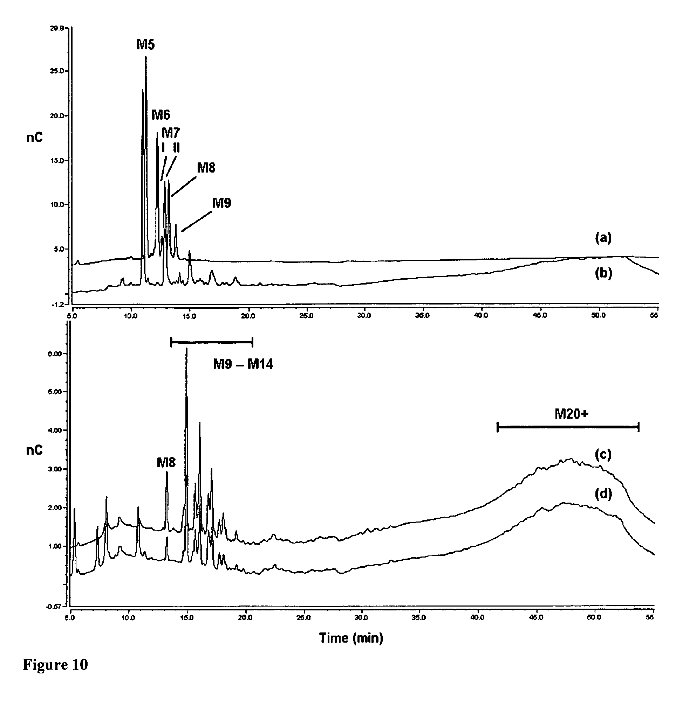

**Figure 10** HPAEC-PAD chromatography of released products purified from (a) Endo H-digested RNase B, where M5 to M9 denote Man₅GlcNAc to Man₉GlcNAc oligosaccharides, (b) WChTv-digested invertase, (c) Endo H-digested invertase, and (d) Endo Tv-digested invertase. M9-M14 and M20+ in the lower panel have been assigned based on the known oligosaccharide structures of invertase (Byrd, et al., 1982; Trimble, et al., 1986) and likely retention times of N-linked oligosaccharides on HPAEC-PAD (Rohrer, 1995).

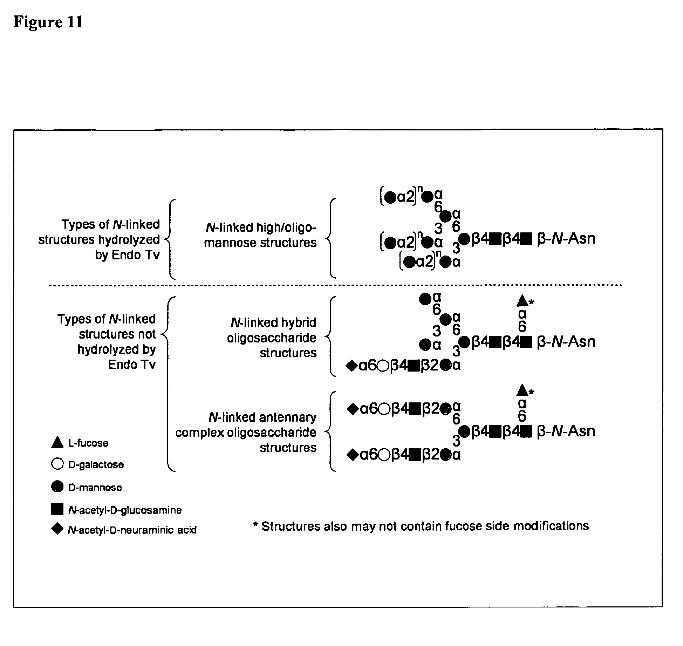

**Figure 11** Types of N-linked structures hydrolysed and not hydrolysed by Endo Tv.

**Table 1.** Specificities of enzymes which cleave *N*-linked oligosaccharides
**Table 2** Relative percentage area of each high mannose oligosaccharide peak (where M5 means Man₅GlcNAc, etc.) released from RNase B by Endo H, WChTv and purified Endo Tv treatment. Relative percentage area is the mean of *n* = 3. The `% difference' column is the percentage increase (+) or decrease (-) of structural isoform released by WChTv or Endo Tv in comparison to that released by Endo H based on relative percentage values. Compared digests were performed, purified and analysed in parallel.
**Table 3.** Glycoproteins tested with WChTv and their *N*-linked oligosaccharide structures.

### Detailed Description of the Drawings

*Materials-* Whole chitinase preparation from *Trichoderma viride,* catalogue C8241 (WChTv) and C6242 (WChTvl), and from Streptomyces griseus (WChSg), catalogue C6137 and fungal protease inhibitor cocktail were purchased from Sigma-Aldrich Co. (Poole, United Kingdom). GlycoClean H cartridges were purchased from Prozyme, Inc. (San Leandro, CA). Labelled lectins ConA and WGA were from EY Laboratories (San Mateo, CA). All other reagents were from Sigma-Aldrich Co. unless otherwise noted and were of the highest grade available. Endo H from *Streptomyces plicatus* is available from Sigma-Aldrich Co. as a recombinant product produced in *E. coli. Trichoderma viride* is available from the ATCC. *Enzymatic digestions of glycoproteins-* Overnight (18 hr) incubations of 1 mg portions of proteins bovine fetuin from fetal calf serum, HRP, and RNase B from bovine pancreas were performed using 6 mU (10 µL) of WChTv which was prepared in 50 mM sodium phosphate, pH 5.0. Enzymatic digestions were treated with 1 mM (final concentration) phenylmethylsufonyl fluoride (PMSF) or 1 µL/mL (final concentration) fungal protease inhibitor cocktail where indicated. Digestions using WChSg and Endo H were essentially as described above with 6 mU and 2 mU of enzyme, respectively. Control blank digests were done at the same time as enzymatic digests and purified and analysed in the same manner. Enzymatic digests which were directly compared with one another were carried out at the same time, i.e. separate Endo H digests were done for comparison to WChTv and Endo Tv digests.

*Sodium dodecyl sulfate-polyacrylamide gel electrophoresis (SDS-PAGE) and lectin blotting-*Treated and non-treated protein samples were mixed with loading buffer containing β-mercaptoethanol and denatured at 100 °C for 5 min. The samples (2 µg each) were electrophoresed on NuPAGE 4-12% Bis-Tris gels using MES running buffer (Invitrogen, Carlsbad, CA) (18) at 150 V constant for approximately 1 h. Gels were stained with 0.05% (w/v) Coomassie G-250 in a fixative solution containing 30% ethanol, and 10% acetic acid and partially destained with distilled deionised water (ddH₂O). Gels were imaged using a ChemiDoc imaging system (Bio-Rad, Hercules, CA).

For blotting experiments, after gel electrophoresis and prior to protein transfer, sequencing grade 0.22 µm polyvinylidene fluoride (PVDF) membrane was washed twice in methanol and rinsed in transfer buffer. Proteins were transferred to PVDF membrane in a semi-dry transfer apparatus (Bio-Rad) at 1.5 mA/cm² for 2 h at a maximum of 15 V. Membranes were probed directly after transfer. Lectin blotting was done using wheat germ agglutinin (WGA) conjugated to alkaline phosphatase as the probe, essentially as previously described (19). In brief, WGA-alkaline phosphatase was used at a concentration of 20 µg in 15 mL lectin buffer (20 mM Tris-HCl, 100 mM NaCl, 0.05% Tween 20, pH 7.4 (TBST), supplemented with 1 mM each of MgCl₂, MnCl₂, and CaCl₂). A control membrane was carried out in parallel and incubated in an identical manner to the lectin blotting experiment except that the lectin was pre-incubated in 100 mM GlcNAc for 1 h prior to, and 10 mM GlcNAc during, the blotting experiment. Lectin binding was visualized colorimetrically by reaction of BCIP/NBT and was stopped by rinsing the membranes in water. The membranes were dried at room temperature in the dark, scanned with white light and stored digitally. *Fluorescently-labelled lectin assay (FLLA)-* FLLA was performed using fluorescein isothiocyanate (FITC)-labeled concanvalin A (Con A) as the probe in a clear-bottomed opaque 96-well microtitre plate. All washes were done three times with TBST, and each assay was performed in triplicate. WChTv-treated and -untreated samples of RNase B were diluted to 125, 100, 50, 25, and 13 µg/mL in alkaline Tris-HCl buffer (50 mM Tris-HCI, pH 10.1). 50 µL of each dilution was used to coat the bottom of a well for 12 h at room temperature with gentle shaking and the protein solution was then aspirated. The wells were washed and blocked with 150 µL 0.5% bovine serum albumin (BSA) in TBST at room temperature for 2 h. The wells were washed, incubated with Con A-FITC (2 µg/mL) for 1 h at room temperature, washed again and read dry at 485 nm excitation and 535 nm emission on a Spectra Fluor microplate reader (Tecan, Männedorf, Switzerland).

*Purification of* endo*-β-N-acetylglucosaminidase (Endo Tv) from WChTv-* The purification of Endo Tv from WChTv was performed on an ÄKTA^{™} Purifier FPLC (GE Healthcare, Uppsala, Sweden). A Mono Q 5/50 anion exchange column (GE Healthcare, USA) was converted to acetate form by sequential washes with 1 M sodium hydroxide (NaOH) for 20 column volumes (CV), 5 CV of deionised water (dH₂O) and 2 CV of 1 N acetic acid. The column was then equilibrated with 5 CV of 5 mM sodium acetate (NaOAc), pH 5.0. 5-8 mg of WChTv was suspended in 500 µL 5 mM NaOAc, pH 5.0, vortexed for 5 min, filtered through a 0.2 µm membrane, centrifuged at 6,000 rpm for 15 min and the supernatant was loaded onto the anion exchange column. A linear gradient from 5 mM to 1 M NaOAc, pH 5.0 was applied over 5 CV at 1 mL/min and elution was monitored at 280 nm. 500 µL fractions were collected. The anion exchange chromatography (AEC) fractions were assayed for glycosidase activity by incubation with RNase B as follows: 15 µL of each fraction was added to 20 µg of RNase B in 50 mM NaOAc, pH 5.0 in a total reaction volume of 80 µL and incubated at 37°C for 2 h. Analysis by SDS-PAGE as described above was carried out to determine whether a shift in the protein band had taken place.

The AEC fraction of interest was further purified by size exclusion chromatography (SEC) on a Superdex 200 10/300 GL column (GE Healthcare) calibrated with IgG (Mr 150 kDa), BSA (Mr 64 kDa), ovalbumin (Mr 45 kDa) and bovine RNase B (Mr 17 kDa). The sample was isocratically eluted with 50 mM NaOAc, pH 5.0 at 250 µL/min. The eluant was monitored at 280 nm and collected in 500 µL fractions and assayed for activity as above. Fractions were pooled appropriately and concentrated by 5 kDa MWCO spin filtration device (Millipore Billerica, MA) and stored at 4 °C.

*Glycosidase activity assays-* Assays for *endo-* and *exo*-chitinase activity were carried out using *para*-nitrophenyl (*p*NP) derivatives of β-*N* acetyl-D-galactosamine, chitobiose, and chitotriose under the following conditions. 2 µL of each AEC or SEC fraction was added to 5 µL *p*NP carbohydrate derivative (6 mM starting concentration), 16 µL dH₂O and 8 µL 100 mM NaOAc, pH 5.0 and incubated for two hours at 37 °C. 20 µL saturated Na₂CO₃ was then added to each reaction, briefly vortexed and 50 µL transferred to a 96 well clear microtitre plate. Absorbance was measured at 405 nm against blank of reaction mixture with 2 µL of dH₂O substituted for enzyme. Assays for β-galactosidase and α-mannosidase activity were conducted in a similar manner with *p*NP derivatives of β-D-galactose and α-D-mannose, respectively.

*Oligosaccharides profiling by high pH anion exchange chromatography with pulsed amperometric detection (HPAEC-PAD)-* Oligosaccharides released from enzymatic digests of various glycoproteins were purified on GlycoClean H cartridges according to manufacturer's instructions. The purified oligosaccharides were then filtered through a 0.22 µm membrane, dried and reconstituted in 100 µL 18.2 MΩ water. The equivalent of 1 µL of the reconstituted oligosaccharide was analysed by HPAEC-PAD on an ICS-3000 system (Dionex, Sunnyvale, CA) equipped with a PA-100 column (4 x 250 mm, Dionex) and PA-100 guard column (4 x 50 mm, Dionex). Oligosaccharides were eluted using a gradient program composed of two buffers, 100 mM NaOH (B) and 1 M NaOAc in 100 mM NaOH (C), at a flow rate of 1 mL/min over 80 min at a column temperature of 25 °C. The linear gradient was started at 5 mM NaOAc in 100 mM NaOH, increased to 50 mM NaOAc in 100 mM NaOH by 10 min and was further increased to 230 mM NaOAc in 100 mM NaOH at 50 min. This molarity was held for 10 min and at 60.1 min the column was washed with 450-500 mM NaOAc in 100 mM NaOH for 7 min. The column was then re-equilibrated at starting conditions from 67.1 to 80 min.

*Liquid chromatography-electrospray ionization mass spectrometry (LC-ESI-MS) oligosaccharide analysis-* The purified oligosaccharides were evaporated to dryness and taken up in 100 µL of HPLC-grade water. 5 µL of each sample was injected onto a 1100 Series HPLC (Agilent, Cork, Ireland) equipped with a Hypercarb porous graphitised carbon column (3 µm, 0.32 x 100 mm) (Thermo Fisher Scientific Inc., UK) and eluted at 7 µL/min using a gradient composed of two buffers: A, 10 mM ammonium carbonate (NH₄HCO₃) and B, 80% acetonitrile in 10 mM NH₄HCO₃. The gradient was linear 0 to 25% B over 45 min, increased to 100% B by 57 min and held for 3 min, decreased to 0% at 61 min and then reequilibrated at 0% B for 20 min before the next injection. Eluate was sprayed into an Agilent LC/MSD Trap XCT mass spectrometer (Agilent) in negative ion mode at a source temperature of 350 °C and needle voltage set at -4300 V.

*Protein identification-* The excised gel band was cut into 1 mm cubes, destained with acetonitrile and reduced and alkylated prior to in-gel trypsin digestion at 37°C based on Shevchenko *et al* (20). 10% formic acid extraction of peptides was followed by concentration on a SpeedVac. NanoLC separation was performed with a PepMap C18 trap and column with the following gradient: 5-35% acetonitrile plus 0.1% formic acid (ACN) over 18 min, 35-50% over 7 min, followed by 95% ACN. Eluate was analysed on a Q-Star Pulsar XL tandem mass spectrometer (Applied Biosystems, Foster City, CA) in Information Dependent Acquisition (IDA) mode. Non-smoothed centroid MS/MS data for doubly and triply charged precursor ions (settings: tolerances of 0.2 Da for precursor and fragment ions, trypsin cleavage, one missed cleavage, fixed carbamidomethyl cysteine modification, methionine oxidation) using Mascot 2.2 (Matrix Science, London, UK) against UniProt (Swiss-Prot and TREMBL combined) April 2009, without species restriction.

### Succinct endo-β-N-acetylglucosaminidase screening methods

Screening for *endo*-β-*N-*acetylglucosaminidase activity in *Trichoderma* growth medium, total lysates prepared from whole *Trichoderma* fungal isolates or protein fractions from either of the above performed by:
1. Incubation of an aliquot of any of the above with glycoproteins containing either high mannose or oligomannose *N*-linked structures using the pH and ionic conditions previously described followed by analysis of the protein or carbohydrate residues after incubation.
2. Incubation of an aliquot of any of the above using the pH and ionic conditions previously described with a fluorescent derivative, colorimetric derivative, radiometric, or reporter enzyme conjugate of either a high mannose or oligomannose structure designed to allow the observation of a resulting portion of the hydrolysis resulting from the incubation.

### RESULTS

*Endoglycosidase activity in* Trichoderma viride *whole chitinase commercial preparation.* The glycoproteins ribonuclease B (RNase B), which has high mannose-type *N*-linked oligosaccharides (Man₅GlcNAc₂ to Man₉GlcNAc₂), and bovine fetuin, which has complex type *N*-linked oligosaccharides as well as *O*-linked oligosaccharides, were treated with a whole chitinase commercial preparation from the fungus *Trichoderma viride* (abbreviated WChTv) and the subsequent observed mass shifts of the glycoproteins on SDS-PAGE were consistent with the loss of *N*-linked oligosaccharides (Fig. 1 (A), lane 2 and (B), lane 2). When compared by SDS-PAGE to Endo F₂- and F₃-digested fetuin, the profile of WChTv-digested fetuin resembled partial deglycosylation or removal of only a specific complex type oligosaccharide(s) (Fig. 1 (A)). However, treatment of fetuin with WChTv in the presence of fungal protease inhibitor cocktail eliminated this change in SDS-PAGE profile (data not shown), which indicated that the previously observed mass shift was due to the removal of an approximately 2 kDa polypeptide from the fetuin by an endogenous protease component of WChTv.

WChTv treatment of RNase B produced a mass shift comparable to treatment of RNase B with the endoglycosidases Endo H and Endo F₁ (Fig. 1), both of which cleave high mannose type *N*-linked oligosaccharides from glycoproteins in an *endo* manner, which was not altered by inclusion of a fungal protease inhibitor cocktail.

A fluorescence-linked lectin binding assay (FLLA) using labelled concanavalin A lectin (Con A) as the probe was performed on RNase B and WChTv-treated RNase B to verify that oligosaccharides were removed from the glycoprotein. Con A binds to α-linked mannose residues and is commonly used to demonstrate the presence of *N*-linked oligosaccharides. The binding of Con A to RNase B was reduced by approximately 80% after treatment with WChTv (Fig. 2 (A)). Wheat germ agglutinin (WGA), which has a binding specificity for *N*-acetylglucosamine (GlcNAc), was used to probe membrane-bound WChTv-treated and -untreated RNase B. Two different WChTv preparation batches (WChTv, as above, and WChTv1) were used to determine if the observed activity was batch specific. WGA bound to the untreated RNase B (Fig. 2 (B), lane 1) and to RNase B treated by both WChTv batches (Fig. 2 (B) lane 2 and 3), which indicated that release action was most likely *endo* and that the oligosaccharide release was not limited to a single batch.

The released oligosaccharides from the WChTv digest of RNase B were purified and analysed by HPAEC-PAD (Fig. 3 (A)). Comparison of the profiles of released oligosaccharides from RNase B by WChTv and Endo H confirmed that the action of the glycosidase was *endo* (Fig. 3 (A) and (B)). The identification of the oligosaccharides released from RNase B by WChTv and Endo H as high mannose-type Man₅GlcNAc to Man₉GlcNAc was confirmed by LC-ESI-MS (data not shown). In addition, the purified products from WChTv, Endo F₂ and Endo F₃ digestions of fetuin were compared by HPAEC-PAD (Fig. 3 (C-E)). No complex-type oligosaccharide release was observed from the WChTv digest of fetuin, although a small peak eluting at the Man₅GlcNAc position was observed (Fig. 3 (C)).

While the activity of WChTv was similar to that of Endo H for the release of high mannose type *N*-linked oligosaccharides from RNase B (Fig. 3 (A) and (B)), the relative percentage, or ratio, of each oligosaccharide released was different (Table 2), which indicated a slightly different structural affinity of the component enzyme(s) of WChTv. Overall, only slightly more Man₇GlcNAc was released from RNAse B by WChTv compared to Endo H (11.4% compared to 11.1%, respectively, Table 2), but the proportions of the structural isoforms were different (28.3% more isomer I (first eluting M7 peak) and 15.4% less isomer II (second eluting M7 peak) was released by WChTv compared to Endo H, Table 2). Man₉GlcNAc release was also decreased (39.5% less was released by WChTv compared to Endo H, Table 2).

The glycoproteins horse radish peroxidase (HRP), bovine immunoglobulin G (IgG), ovalbumin and alcohol dehydrogenase were also incubated with WChTv and analyzed by SDS-PAGE to test for potential release of oligosaccharides (see Table 3 for results). RNase B and ovalbumin samples were also treated with whole chitinase preparation from *Streptomyces griseus* (WChSg), a Gram-negative bacterium. No SDS-PAGE migration shift was apparent in the case of RNase B (Fig. 4 (A)), indicating a lack of oligosaccharide release, but a migration shift of a small proportion of ovalbumin was noted (Fig. 4 (B)). However, no significant peaks were noted in the HPAEC-PAD chromatograph in comparison to oligosaccharides released from ovalbumin by WChTv or Endo H (Fig. 4 (C)), so the quantity may have been quite low.

*Purification of novel endo-β-N-acetylglucosaminidase from WChTv.* WChTv was resuspended in 5 mM NaOAc, pH 5.0 and the Bradford estimation indicated that the protein content of the solid starting material was approximately 22.9% of the total weight. After filtration, the solution retained only half of the original protein content (11.8%). While this reduction corresponds to a substantial loss in measured protein content, a comparison of activity between filtered and unfiltered WChTv solution did not show a noticeable reduction in RNase B deglycosylation activity (data not shown).

AEC of the filtered, soluble protein WChTv resulted in multiple peaks (Fig. 5), which were screened for endoglycosidase activity by observing a mass shift by SDS-PAGE of RNase B after incubating with a portion of each fraction. No glycosidase activity was apparent in the flow-through or wash elution fractions. Only those fractions which eluted between 195 to 295 mM NaOAc demonstrated glycosidase activity, which indicated either multiple *endo*-β-*N*-acetylglucosaminidases or multiple isoforms of one *endo*-β-*N-*acetylglucosaminidase. The well defined peak at approximately 295 mM NaOAc (fraction C1, Fig. 5) contained the most potent activity and was therefore selected for further purification. SDS-PAGE of the AEC fractions themselves demonstrated multiple protein components in the majority of fractions (Fig. 6).

The AEC C1 fraction was further purified by SEC, eluted isocratically with 50 mM NaOAc, pH 5.0. At this molarity and pH, the stability and solubility of the protein was consistent. The collected peaks 1 (which corresponded to a native mass of approximately 50 kDa), 2 (approximately 35 kDa) and 3 (approximately 20 kDa) (Fig. 7 (A)) demonstrated endoglycosidase activity when tested with RNase B. The highest activity was from peak 2 (Fig. 7 (B)) and as peak two overlapped with peaks 1 and 3, the lower activity from these peaks was attributed to a lower concentration of enzyme from peak 2 (Fig. 7 (A)). Peaks 4-6 did not show endoglycosidase activity with RNAse B.

SDS-PAGE analysis of peaks 1, 2 and 3 was performed under reducing (R) and nonreducing (NR) conditions (Fig. 7 (C)) and observed masses for the major bands in the reduced SDS-PAGE gel corresponded well with masses estimated from SEC e.g. peak 2 was found to have a Mr of 33 kDa which compared well with the 35 kDa estimated by SEC. In the NR-SDS-PAGE lanes, some higher mass complexes were apparent with protein from peaks 1 and 2 which are typical of dimer formation. No protein could be seen from peak 3 under either R or NR conditions, indicating that the quantity of protein present was below the sensitivity threshold of Coomassie G250 staining. The total protein content of peak 2 was estimated as 2 µg, which corresponded to 0.31% of the original WChTv protein content. *Endo-* and *exo*-chitinases from *T. viride* are reported at Mr 64, 42 and 25 kDa. Peak 1 demonstrated chitinase activity, which corresponded well with the mass of 42 kDa. Peak 2 had a small % of the activity of peak 1 which was attributed to overlap of peak 1 and 2. *Protein identification of purified* T. viride *enzyme.* In-gel trypsin digestion of the 35 kDa band from SEC peak 2 and subsequent nanoLC-MS/MS resulted in the acquisition of several peptide sequences. However, subsequent BLAST analysis of non-redundant protein sequence database from the National Centre for Biological Information (NCBI) gave only a single identity match for the peptide AEPTDLPR (observed mass 449.73 Da) with *endo*-β-*N* acetylglucosaminidase from *T. reesei* (Fig. 8). BLAST (BLASTp) produced potentially homologous matches for two additional peptide masses, EPARLIAR (observed mass 463.32 Da), and LLAVVLSTLLVFGFAPVAK (observed mass 979.70 Da). However, the latter sequence was split when aligned with the *endo*-β-*N*-acetylglucosaminidase from *T. reesei* (Fig. 8). The mass reported for the *T. reesei* enzyme (33 kDa) is approximately that observed by chromatographic and PAGE size estimation for the intact *endo*-β-*N*-acetylglucosaminidase from species *T. viride* (Endo Tv).

*Characterisation of purified novel Endo Tv activity.* Although multiple structural isoforms of each oligosaccharide exist (25), high-mannose *N*-linked oligosaccharides with the same mannose number mainly elute in the same peak by HPAEC-PAD, except in the case of Man₇GlcNAc and Man₈GlcNAc, where oligosaccharides eluted in two separate peaks ((26) and Fig. 9(a)). We were unable to attribute a small peak between those attributed to Man₅GlcNAc and Man₆GlcNAc and hence this was designated M5/6 (Fig. 9(a) and Table 2). After purification of the novel enzyme Endo Tv from WChTv, the proportions of high-mannose N-linked oligosaccharides released from RNase B by Endo Tv were altered as compared to those released by WChTv (Fig. 9 (b) and Table 2). There was an increase in the relative percentage release of Man₅GlcNAc and Man₇GlcNAc isomer I and a decrease in the proportion of Man₈GlcNAc isomer I (see Table 2).

Interaction(s) with other component(s) in the WChTv may have inhibited the release of other isomers of Man₅GlcNAc and Man₇GlcNAc prior to Endo Tv purification or indeed a combination of enzymes may have been responsible for the oligosaccharide release using WChTv. In addition to the expected *endo-* and *exo*-chitinase activities of WChTv, α-mannosidase and *exo*-a-*N*-acetylgalactosminidase activities were also observed using the appropriate *p*NP-derivative substrates (data not shown), which lends support to the latter possibility. In the case of Man₈GlcNAc, it is possible another *endo*-β-*N-*acetylglucosaminidase component of WChTv was responsible for this action, especially taking into account the observation of less some low-level *endo*-β-*N-*acetylglucosaminidase activity observed in additional FPLC peaks which were not further purified.

In comparison of oligosaccharides released from RNase B, Endo Tv released proportionately more Man₅GlcNAc and Man₇GlcNAc isomer I and less of Man₈GlcNAc isomer I and Man₉GlcNAc than did Endo H (Table 2).

Additional egg white glycoproteins are usually co-purified with commercial ovalbumin, and high-mannose, hybrid and bisecting GlcNAc *N*-linked structures from purified and co-purified glycoproteins have been reported as from commercial ovalbumin (27). Of interest to this work, the high-mannose type structures ManGlcNAc₂, Man₂GlcNAc₂, Man₃GlcNAc₂ (28), Man₄GlcNAc₂, Man₅GlcNAc₂, Man₆GlcNAc₂, Man₇GlcNAc₂ (27) and Man₈GIcNAC₂ (29,30) have all been reported in ovalbumin or commercial preparations of ovalbumin. Endo Tv released only high-mannose structures from ovalbumin including ManGlcNAc, Man₃GlcNAc and Man₅GlcNAc, Man₆GIcNAc and Man₈GlcNAc (Fig. 9 (d)). When fetuin was used as a substrate, a small amount of Man₅GlcNAc release was observed upon treatment with Endo Tv (Fig. 9 (e)). Man₃GlcNAc₂ to Man₇GlcNAc₂ have been reported as minor components of HRP, in addition to the more abundant xylosylated and fucosylated structures (31,32). Minor peaks corresponding to Man₃GlcNAc and Man₇GlcNAc were observed after Endo Tv digestion of HRP (Fig. 9 (f)), in addition to several unidentified minor peaks which eluted later than Man₉GlcNAc. However, these peaks from the HRP digests were also observed from control digestion sample which had no enzyme added, so these oligosaccharides must have been present free in the glycoprotein preparation and were not released from HRP by the action of the enzyme.

*N*-linked oligosaccharides of yeast glycoproteins are exclusively of the high mannose type. Inverstase from *Saccharomyces cerevisiae* has been reported to have two main size distributions of high mannose oligosaccharides: Man₈GlcNAc₂ to Man₁₄GlcNAc₂ and Man>₂₀GlcNAc₂ (33,34). Similar HPAEC-PAD profiles were observed for oligosaccharides released from yeast invertase by Endo H and Endo Tv (Fig. 10 (c) and (d)), with several additional peaks eluted before Man₈GlcNAc in the Endo Tv-treated invertase digest. A different profile was observed when invertase was treated with WChTv (Fig. 10 (b)), supporting the previous observation of multiple enzymatic components of the WChTv extract which acted in concert in this digestion.

### DISCUSSION

This is the first report of an *endo*-β-*N*-acetylglucosaminidase from *Trichoderma viride.* The purified novel enzyme, Endo Tv, released Man₅GlcNAc to Man₉GlcNAc oligosaccharides from RNase B, ManGlcNAc, Man₃GlcNAc, Man₅GlcNAc, Man₆GlcNAc and Man₈GlcNAc from ovalbumin, and high mannose structures from yeast invertase, but no oligosaccharides were released from HRP. The enzyme does not appear to act on fucosylated, xylosylated, hybrid or complex-type *N*-linked oligosaccharides.

The genus *Trichoderma* are a broad group comprised primarily of soil-dwelling, filamentous fungi and are widely exploited for their copious production of industrially-important extracellular hydrolases. In particular, *Trichoderma* species are known to be sources of potent cellulose-degrading enzymes. Under specific conditions, enzymes from *T*. *viride* previously have been shown to exhibit additional activities than those described during original characterizations. For example, in addition to producing a separate β-xylosidase (35) at lease one cellulase from *T. viride* also cleaves the xylosyl-serine linkage between a glycosaminoglycan (GAG) chain and the core protein (36).

Judging by the many components visible in the SDS-PAGE of anion exchange separation of WChTv (Fig. 8, lane W), the 'chitinase' would be better described as a *T. virile* extract which includes chitinases among other enzymes and components. In addition to the expected *endo-* and *exo*-chitinase activites of WChTv, α-mannosidase and *exo*-α-*N-*acetylgalactosaminidase activities were also observed using the appropriate *p*NP-derivative substrates (data not shown). Bovine fetuin contains a high amount (∼82%) tri-antennary *N-*linked structures which terminate in sialylated *N*-acetyllactosamine (LacNAc). Although a mass shift of fetuin was observed by SDS-PAGE upon treatment with WChTv, further investigation revealed that there were no complex oligosaccharides released from fetuin and that the loss of mass in the glycoprotein was due to protease action.

HPAEC-PAD analysis did, however, indicate the presence of a minute quantity of Man₅GlcNAc from the 1 mg fetuin sample treated with purified EndoTv (Fig 11 (e)). This may indicate presence of a small very small percentage of fetuin containing incompletely processed N-linked oligosaccharides. Based on the absence of a peak corresponding to the Man₅GlcNAc structure in the chromatograph from the Endo Tv-treated HRP sample (Fig 11(f)), as well as the absence of the same peak in control samples containing only the EndoTv enzyme, the Man₅GlcNAc peak present in the Endo Tv treated fetuin sample was not introduced by the Endo Tv enzyme component itself and therefore must have been released from a subpopulation of the fetuin sample.

Trypsin-like protease activity from *T. viride* has previously been reported (41). Therefore, *T. viride* chitinase preparation is generally unsuitable for use without further purification to isolate additional enzymatic components, particularly if protein and/or carbohydrate components are the subject of investigation. However, in this work the protease activity of WChTv was controlled by the addition of a fungal inhibitor cocktail which did not have a marked reduction on the ability of WChTv to cleave oligosaccharides from RNase B.

In addition to being important in structure-function studies, the discovery of Endo Tv in a commercial chitinase preparation which is widely in use may also have important implications for understanding atypical phenomena previously reported which implied *in vivo* presence of chitin-containing structures in unlikely sources. For example, the increased electrophoretic migration of human ocular mucins after chitinase treatment (42) is more likely as a result of the effect of *N*-linked oligosaccharide cleavage, release of GAG chains by a secreted cellulase or β-xylosidase and/or partial protease degradation from additional components in the enzyme preparation rather than the presence of chitin in mucin.

In this work, a chitinase preparation from the bacterium *Streptomyces griseus* (WChSg) was also tested for endoglycosidase activity. No band migration for RNase B was observed by SDS-PAGE after WChSg treatment (Fig. 5 (A)), but a small proportion of ovalbumin migrated to a lower mass (Fig. 5 (B)). However, the presumed released oligosaccharides were not detectable by HPAEC-PAD (Fig. 5 (C)), so this shift may have been due to the action of a protease component of WChSg.

*Elizabethkingia meningoseptica,(43)* formerly known as *Chryseobacterium meningosepticum* and prior to that as *Flavobacterium meningosepticum,* is the source of the amidase PNGase F (EC 3.5.1.52) and the endoglycosidases Endo F₁, F₂ and F₃ (see Table 1 for specificities). The substrate specificities of Endo F₁, F₂ and F₃ were reported based on oligosaccharide cleavage from glycopeptides or oligosaccharides (6,44-46), and further, porcine fibrinogen glycopeptides have been recommended as ideal substrates for assaying the activity of Endo F₂ and F₃. Despite the necessity of some form of denaturation of many glycoproteins to achieve deglycosylation using Endo F₂ and F₃, these enzymes are usually included in commercial kits for native protein deglycosylation. In agreement with previous reports, we found recombinant Endo F₂ and F₃ to be not very effective on native fetuin. It is clear that additional endoglycosidases which have good activity on native glycoproteins are required for the glycobiologist's 'toolkit'.

Based on the identified peptide fragment sequences derived from the *T. viride endo*-β-N-acetylglucosaminidase and the reported full-length sequence data for the *endo*-β-*N-*acetylglucosaminidase from *T. reesei* (16), these enzymes both may potentially be assigned to the glycosyl hydrolase 18 family. However, unlike the bacterial endoglycosidases F₁ and H, which share high degrees of conserved sequence with each other, these two examples of *Trichoderma* endoglycosidases do not share homology with the bacterial *endo*-*N-*acetylglucosaminidases, but instead share more homology with chitinases.

While other *endo*-β-*N-*acetylglucosaminidases have been discovered and are in common use in glycobiology, e.g. Endo H and Endo F₁ (5,21), the preference of Endo Tv for certain Man₅GlcNAc to Man₉GlcNAc structural isoforms is different from the preference of Endo H (45, 25, 14, 11 and 3% compared to 37, 27, 11, 16 and 8%, respectively, for Man₅GlcNAc to Man₉GlcNAc oligosaccharides released from RNase B). The change in the relative ratio of high-mannose oligosaccharides released from RNase B (Table 2) after purification of Endo Tv from WChTv may indicate that additional *endo*-β-*N-*acetylglucosaminidase(s) were present in WChTv, which is supported by the observation of *endo*-β-*N-*acetylglucosaminidase activity present in other AEC fractions (Fig. X?).

Fungi are known to produce high mannose and oligomannose N-linked oligosaccharide structures. The secrection of an enzyme capable of releasing high-mannose N-linked structures often found in fungal species is consistent with *Trichoderma* mycoparasitism. High-mannose structures are also found in the plant and animal kingdoms, although differences in the occurrence of certain structural isoforms has been reported. Even though the preference of rice and tomato *endo*-β-*N*-acetylglucosaminidases for certain high mannose structures typically found in plants is known (54-56), this is the first comparison of preferences for certain oligosaccharide structural isoforms of *endo*-β-*N-*acetylglucosaminidases between species (filamentous fungi and Gram-negative bacteria) and consequently may prove useful in future structure-function studies.

In addition, it is clear from this work that the source of the enzyme chosen, if not the individual enzyme itself, may have an influence on the distribution of the structural isoforms of the oligosaccharides released from the glycoprotein under investigation. Hence, the released population analysed may be otherwise biased compared to the actual population present. Therefore, a comparison of the population released with the population released by an enzyme with the same function but from another source would be beneficial in determining whether such a bias has been introduced and help establish the true distribution of structural isoforms present on the glycoprotein.

### CONCLUSION

A commercial preparation of poly β-*N*-acetylglucosamine hydrolase (chitinase) from the fungus *Trichoderma viride* (*Hypocrea rufa*) effectively hydrolyzed high-mannose-type *N-*linked oligosaccharides from RNase B under non-denaturing conditions. Separation of the whole preparation by anion exchange chromatography and subsequent assays of the fractions with *p*NP-GlcNAc, *p*NP-chitotriose and RNAse B substrates indicated that the component responsible for glycosidase activity was distinct from reported *T. viride* chitinases. Purification to a single active component by size exclusion chromatography resulted in a single enzyme with approximate molecular mass of 35 kDa. In-gel trypsin digestion and mass spectrometry (MS) of the protein band indicated homology to an *endo*-β-*N* acetylglucosaminidase from *T. reesei.* The action of the purified enzyme was determined to be *endo* by analysis of the purified released carbohydrates by LC-MS and high pH anion exchange chromatography with pulsed amperometric detection (HPAEC-PAD). The purified *T. viride* enzyme was able to cleave high mannose type oligosaccharides from RNase B, ovalbumin, and yeast invertase, but did not release hybrid or complex-type structures, nor did it have any activity on fucosylated or bisecting *N*-acetylglucosamine (GlcNAc)-bearing structures. The novel *endo*-β-*N-*acetylglucosaminidase from *T. virde* (Endo Tv) is thus similar in action to Endo F₁ and Endo H, although the protein sequence of Endo Tv is highly dissimilar to the bacterial versions. Interestingly, a different ratio of high mannose type oligosaccharide structural isoforms was released from RNase B by Endo Tv compared to Endo H. Endo Tv may therefore prove valuable in structure-function relationships. In addition, the use of another enzyme in glycoprotein *N*-linked oligosaccharide structural studies may serve to balance a bias in structural isoforms in released populations unintentionally introduced by the use of an enzyme from only one source.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

**Table 1. Specificities of some of the enzymes used to remove N-linked oligosaccharides from glycoproteins.**

| **Enzyme** | **EC** | **Source organism** | **Enzyme class** | **Specificity** | **Reference** |
|---|---|---|---|---|---|
| PNGase F | 3.5.1.52 | *E. meningosepticu m* | Amidase | Complex, hybrid, high mannose, α-(1→4,6) fucosylation only | (Plummer, T.H., Jr. and Tarentino, A.L. 1991) |
| PNGase A | 3.5.1.52 | *P. dulcis* | Amidase | Complex, hybrid, high mannose, α-(1→3, 4,6) fucosylation | (Altmann, F., Paschinger, K., et al. 1998) |
| Endo D | 3.2.1.96 | *S*. *pneumoniae* | Endoglycosidase | Trimannosyl, non-sialylated complex | (Muramatsu, H., Tachikui, H., et al. 2001) |
| Endo H | 3.2.1.96 | *S*. *plicatus* | Endoglycosidase | High mannose, α-(1→3,4,6) fucosylation | (Trimble, R.B. and Maley, F. 1984) |
| Endo S | 3.2.1.96 | *S*. *pyogenes* | Endoglycosidase | Complex biantennary, α-(1→6) fucosylation | (Collin, M. and Olsen, A. 2001) |
| Endo F1 | 3.2.1.96 | *E. meningosepticu m* | Endoglycosidase | Hybrid, high mannose | (Tarentino, A.L., Quinones, G., et al. 1992) |
| Endo F2 | 3.2.1.96 | *E*. *meningosepticu m* | Endoglycosidase | High mannose, biantennary complex | (Reddy, A., Grimwood, B.G., et al. 1998) |
| Endo F3 | 3.2.1.96 | *E. meningosepticu m* | Endoglycosidase | Trimannosyl, biantennary and triantennary complex, fucose position dependent | (Tarentino, A.L. and Plummer, T.H., Jr. 1994) |

**Table 2 Relative percentage area of each high mannose oligosaccharide peak (where M5 means Man5GlcNAc, etc.) released from RNase B by Endo H, WChTv and purified Endo Tv treatment. `% difference' column is the percentage increase (+) or decrease (-) of structural isoform released by WChTv or Endo Tv in comparison to that released by Endo H based on relative percentage values. Compared digests were performed, purified and analysed in parallel.**

| **Structures** | **Relative % released** | | **% difference** | **Relative % released** | | **% difference** |
|---|---|---|---|---|---|---|
| | **Endo H** | **WChTv** | | **Endo H** | **Endo Tv** | |
| M5 | 36.7 | 39.8 | 8.4 (+) | 37.6 | 45.2 | 20.2 (+) |
| M5/6 | 1.3 | 0.8 | 38.5 (-) | 1.3 | 2 | 53.8 (+) |
| M6 | 26.5 | 27.2 | 2.6 (+) | 26.4 | 24.7 | 6.4 (-) |
| M7 I | 4.6 | 5.9 | 28.3 (+) | 4.3 | 7.6 | 76.7 (+) |
| M7 II | 6.5 | 5.5 | 15.4 (-) | 6.2 | 6 | 3.2 (-) |
| M8 I | 15.3 | 15.1 | 1.3 (-) | 15.7 | 10.5 | 33.1 (-) |
| M8 II | 1.1 | 0.8 | 27.3 (-) | 0.9 | 0.8 | 11.1 (-) |
| M9 | 8.1 | 4.9 | 39.5 (-) | 7.7 | 3.3 | 57.1 (-) |

**Table 3. Glycoproteins tested with WChTv and their N linked oligosaccharide structures.**

| **Glycoprotein** | **SDS-PAGE alteration** | **Source Organism** | **Most Prevalent N-linked Oligosaccharides** |
|---|---|---|---|
| Fetuin | Yes* | *B. taurus* | Bi-, tri- and tetra-antennary complex |
| RNase B | Yes | *B. taurus* | High mannose |
| Ovalbumin | Yes | *G. gallus* | High mannose, hybrid, bisecting GlcNAc |
| IgG | No | *B. taurus* | Biantennary complex, bisecting GlcNAc, α(1,6)-linked fucose |
| Invertase | Yes | *S*. *cerevisiae* | High mannose, yeast-type high-mannose |
| HRP | Yes* | *A. rusticana* | Trimannosyl, β-(1,2)-linked xylose, α-(1,3)-linked fucose |

| | | | |
|---|---|---|---|
| * Later attributed to protease activity. See text for futher details | | | |

### REFERENCES

5. Trimble, R. B., and Maley, F. (1984) Analytical biochemistry 141(2), 515-522
6. Trimble, R. B., and Tarentino, A. L. (1991) The Journal of biological chemistry 266(3), 1646-1651
18. Laemmli, U. K. (1970) Nature 227(5259), 680-685
19. Kilcoyne, M., Shah, M., Gerlach, J. Q., Bhavanandan, V., Nagaraj, V., Smith, A. D., Fujiyama, K., Sommer, U., Costello, C. E., Olszewski, N., and Joshi, L. (2009) Journal of Plant Physiology 166(3), 219-232
20. Shevchenko, A., Wilm, M., Vorm, O., and Mann, M. (1996) Analytical chemistry 68(5), 850-858
27. Harvey, D. J., Wing, D. R., Kuster, B., and Wilson, I. B. H. (2000) Journal of the American Society for Mass Spectrometry 11(6), 564-571
28. Nomoto, H., Endo, T., and Inoue, Y. (1982) Carbohydrate research 107(1), 91-101
29. Suzuki, T., Kitajima, K., Emori, Y., Inoue, Y., and Inoue, S. (1997) Proceedings of the National Academy of Sciences of the United States of America 94(12), 6244-6249
30. Anumula, K. R., and Dhume, S. T. (1998) Glycobiology 8(7), 685-694
31. Yang, B. Y., Gray, J. S. S., and Montgomery, R. (1996) Carbohydrate research 287(2), 203-212
32. Gray, J. S. S., Yang, B. Y., and Montgomery, R. (1998) Carbohydrate research 311(1-2), 61-69
33. J.C. Byrd, A.L. Tarentino, F. Maley, P.H. Atkinson, and R.B. Trimble, Glycoprotein synthesis in yeast. Identification of Man8GlcNAc2 as an essential intermediate in oligosaccharide processing. J. Biol. Chem. 257 (1982) 14657-14666.
34. R.B. Trimble, and P.H. Atkinson, Structure of yeast external invertase Man8-14GlcNAc processing intermediates by 500-megahertz 1H NMR spectroscopy. J. Biol. Chem. 261 (1986) 9815-9824.
41. Uchikoba, T., Mase, T., Arima, K., Yonezawa, H., and Kaneda, M. (2001) Biological chemistry 382(10), 1509-1513
42. Argüeso, P., Herreras, J. M., Calonge, M., Citores, L., Pastor, J. C., and Girbés, T. (1998) Cornea 17(2), 200-207
43. Kim, K. K., Kim, M. K., Lim, J. H., Park, H. Y., and Lee, S.-T. (2005) Int J Syst Evol Microbiol 55(3), 1287-1293
44. Tarentino, A. L., Quinones, G., Changchien, L. M., and Plummer, T. H., Jr. (1993) The Journal of biological chemistry 268(13), 9702-9708
45. Tarentino, A. L., and Plummer, T. H., Jr. (1994) Methods in enzymology 230, 44-57
46. Tarentino, A. L., and Plummer, T. H., Jr. (1994) Glycobiology 4(6), 771-773

## Claims

**1.** An *endo*-β-*N*-acetylglucosaminidase enzyme isolatable from the fungus *Trichoderma virde* **characterised in that**:-
c) the enzyme has an approximate molecular mass of 35 kDa;
d) the enzyme is *endo*-acting;
c) the enzyme can cleave high mannose type *N*-linked oligosaccharides from native bovine RNase B, ovalbumin and yeast invertase, at pH 4.5 to 7 under non-denatruing conditions.
f) the enzyme does not release *N*-linked oligosaccharides containing extended, non-mannosyl residues (i.e. complex) or *N*-linked oligosaccharides containing extended non-mannosyl residues in conjunction with mannosyl extended structures (i.e. hybrid);
g) the Endo Tv enzyme has no activity on fucosylated or bisecting *N*-acetylglucosamine (GlcNAc)-bearing structures.

**2.** An enzyme as claimed in claim 1 which has no activity on fucosylated or bisecting *N-*acetylglucosamine (GlcNAc)-bearing structures.

**3.** An enzyme as claimed in claim 1 or 2 which, on trypsin digestion, releases all of the following peptides :-
AEPTDLPR, EPARLIAR and LLAVVLSTLLVFGFAPVAK.

**4.** An enzyme as claimsed in any preceding claim **characterised in that** it results in an increase in the relative percentage release of Man₅GlcNAc and Man₇GlcNAc isomer I and a decrease in the proportion of Man₈GlcNAc isomer I, from RNase B, as compared to the structural isoforms released by WChTv.

**5.** An enzyme as claimed in any preceding claim **characterised in that** it release proportionately more Man₅GlcNAc and Man₇GlcNAc isomer I and less of Man₈GlcNAc isomer I and Man₉GlcNAc than does Endo H.

**6.** An enzyme as claimed in any preceding claim **characterised in that** it is isolatable from whole chitinase preparation from *Trichoderma viride* (WChTv) or from the medium in which *Trichoderma viride* has been cultured..

**7.** An enzyme as claimed in any preceding claim **characterised in that** it has no activity on fucosylated or bisecting *N*-acetylglucosamine-bearing *N*-linked oligosaccharide structures, such as those from ovalbumin, xylosyl side modifications, such as those from horseradish proxidase, or upon complex structures such as from fetuin and immunoglobulin G.

**8.** A method for the purification of *endo*-β-*N-*acetylglucosaminidase by any combination of ion exchange chromatography, size exclusion chromatography, hydrophobic interaction chromatography and ammonium sulphate precipitation.

**9.** A method as claimed in claim 8 comprising weak ion exchange chromatography followed by strong ion exchange and size exclusion chromatography.

**10.** A method as claimed in claim 8 or 9 comprising:-
(a) solubilisation of a whole chitinase preparation from *Trichoderma viride* in an aqueous buffer;
(b) purification of the solubilised preparation by size exclusion techniques;
(c) selection of at least one fraction which elutes between 195 and 295 mM NaOAc
(d) further purification of the selected fraction produced in step (c) by size exclusion chromatography with a salt buffer at pH 4.5 - 7.

**11.** A method as claimed in claim 10 wherein the aqueous buffer for solublisation is NaOAc.

**12.** A method as claimed in claim 10 or 11 wherein the salt buffer of step (d) is NaOAc.

**13.** A method as claimed in claim 9 wherein the enzyme is isolated by filtration and sequential step liquid chromatography of the secreted protein mixture contained in the culture medium *Trichoderma virde.*

**14.** An *endo*-β-*N-*acetylglucosaminidase whenever prepared by a process claimed in any of claims 9 to 13.

**15.** Use of an enzyme as claimed in any one of claims 1 to 8 or claim 14 to remove oligomannose (M>9) and high-mannose structures (M1-9) from glycoprotein drug products.

**13.** Use as claimed in claim 15 where in the glycoprotein drug product has been produced from yeast.

**14.** Use of an enzyme as claimed in any one of claims 1 to 8 or claim 14 for the quantitative and qualitative analysis of oligomannose and high-mannose components of glycoprotein samples, to release glycans from fungal glycoproteins, in a method of verifying the presence of oligomannose and high-mannose structures on glycoprotein, for the removal of mannose from biomaterials, to increase the half life of molecules, in the synthesis of products by transferase to make glycoprotein structures, to generate high mannose molecules for CDG treatment, in the production of therapeutics, nutraceuticals, and in the synthesis of reagents for drug production and analysis.
